# EUROPEAN PATENT SPECIFICATION

(11) **EP 3 027 734 B1**
(45) Date of publication and mention of the grant of the patent: **25.10.2017**
(21) Application number: 14752554.7
(22) Date of filing: 30.07.2014
(51) Int. Cl.: C12N 1/20, A23K 30/18, A23K 50/10, C12R 1/25

(54) **ISOLATED MICROORGANISM STRAIN LACTOBACILLUS PLANTARUM TAK 59 NCIMB42150 AND ITS USE**
LACTOBACILLUS PLANTARUM STAMM TAK 59 NCIMB42150 UND DESSEN VERWENDUNG
SOUCHE DE LACTOBACILLUS PLANTARUM TAK 59 NCIMB42150 ET SON UTILISATION

(30) Priority: 30.07.2013 EE 201300027
(43) Date of publication of application: 08.06.2016
(73) Proprietor: Oü Tervisliku Piima Biotehnoloogiate Arenduskeskus, 51014 Tartu (EE)
(72) Inventor: OLT, Andres, EE-50407 Tartu (EE); KALDMÄE, Helgi, EE-51006 Tartu (EE); KÄRT Olav, EE-13516 Taééonn (EE); OTS, Meelis, EE-51006 Tartu (EE); SONGISEPP, Epp, EE-50107 Tartu (EE); RÄTSEP, Merle, EE-50109 Tartu (EE); KOKK, Kristiina, EE-61715 Tartumaa (EE); STSEPETOVA, Jelena, EE-50606 Tartu (EE); KÕLJALG, Siiri, EE-50404 Tartu (EE)
(74) Representative: Kahu, Sirje
(86) International application number: PCT/EE2014/000003
(87) International publication number: WO 2015/014372

(56) References cited:
- EP-A1- 1 386 543
- EP-A2- 0 580 236
- WO-A1-2011/159178
- E. SAARISALO ET AL: "Screening and selection of lactic acid bacteria strains suitable for ensiling grass", JOURNAL OF APPLIED MICROBIOLOGY, vol. 102, no. 2, 1 February 2007 (2007-02-01), XP055146502, ISSN: 1364-5072, DOI: 10.1111/j.1365-2672.2006.03103.x

## Description

### TECHNICAL FIELD

The invention belongs to the field of biotechnology and is applied to feed manufacturing. The invention encompasses a microbiological silage additive and its use in feed fermentation in order to increase the quality of fermentation and of the feed.

### BACKGROUND ART

In precision livestock farming today with high milk production it is crucial to ensure that animals get high quality feed all year round. Silage has proven to be such a feed. Silage is the material produced by the controlled fermentation of a crop of high moisture content (McDonald, P., Henderson, A. R., Heron, S.J.E. 1991. The biochemistry of silage. 2nd ed. Chalcombe Publications, Marlow, Bucks UK, p. 340). In addition to grass and legume silage forages, cereals, whole-crop cereals (incl. maize), by-products (i.e. wet distillers grain, brewers' grains, pulp) of alcohol and sugar industry can also be fermented to provide feed.

Ensiling is a complex of several different chemical and microbiological processes and of their concurrence. The natural fermentation of feed is hard to control. However, ensiling is considered to be lactic acid fermentation with no oxygen (Rooke, J., A. and Hatfield, G., D., 2003. Biochemistry of Ensiling. In: Silage Science and Technology. D. R. Buxton, R. E. Muck, and J. H. Harrison, eds. American Society of Agronomy, Madison, Wisconsin, USA. pp. 95-139). This means that in anaerobic conditions, micro-organisms (ideally lactic bacteria) ferment watersoluble carbohydrates into organic acids. Under favourable conditions, the fermentation process predominantly produces lactic acid, and as a result the pH of the silage starts to decrease. This decrease in pH inhibits the growth and action of undesirable microorganisms, and hence the feed is preserved. The faster the pH of silage decreases to a pH of 4, the faster the feed stabilises and the more nutrients can be preserved.

The success or difficulty of ensiling depends on the quality of the plant material that is being ensiled, and on the technological methods used in the production of the silage. It is difficult to ensile plant material with a low dry matter content, and water-soluble carbohydrates and also with plant material that has a high buffering capacity (Pahlow, G., Rammer, C., Slottner, D., Tuori, M. Ensiling of legumes. In: Wilkins R. J. and Paul, C. (eds): Legumes Silages for Animal Production - LEGSIL. Braunschweig, FAL, 2002, pp. 27-31). According to the European Union Commission Regulation (EC) No 429/2008 of 25 April 2008, it is considered difficult to ensile forage that has a <1.5 per cent water-soluble carbohydrates (WSC) content, easy to moderately difficult to ensile forage that has from 1.5-3 per cent WSC, and it is easier still if the material has a higher than 3 per cent WSC content. In addition to this, the weather conditions also affect ensiling and the quality of silage. With sub-optimal weather conditions, it is not possible to wilt the plant material, in order to increase the dry matter content and the WSC content and to decrease the buffering capacity, i.e. to increase the ensilability of the plant material. Difficult ensiling conditions also favour the development of undesirable microorganisms (enteropathogens, clostridia, bacilli, fungi, listeria) that causes spoilage of feed. Alas, one cannot choose harvesting weather during the optimal developing stage of silage crops. The chemical content of silage depends mostly on the plant development stage and the quality of the fermentation of the plant material. Silage with low nutrient value, which is made of plants harvested at a late stage of growth, reduces the productivity of the animals feeding on it, yet spoilt feed cannot be fed to animals. In evaluating the quality of silage, fermentation parameters are crucial. The more parameters there are to evaluate the success of ensiling, the better the overview of the quality of the feed. The most important fermentation parameters (not listed in order of importance) are: the concentrations of ethanol, volatile fatty acids (acetic, propionic, butyric acid) and lactic acid, the pH and the ammonia nitrogen content in total nitrogen. The kinds of fermentation products that are generated depend on the silage crop, on the technological methods used in the production of the silage, on the population of microorganisms in the ensiling material at storage, and on the air-tightness of the storage.
Silage additives are used to positively affect the ensiling process. Silage additives are understood to be additives that improve lactic acid fermentation and accelerate the decrease in pH during ensiling and/or inhibit the action of undesirable microorganisms in the plant material at storage, and hence avoide damaging products produced by them.

The most common silage additives are enzymes, sugars, acids, salts and bacteria inoculants. (Kung, L., Stokes, M. R., Lin, C. J., 2003. Silage additives. In: Silage Science and Technology. D. R. Buxton, R. E. Muck, and J. H. Harrison, eds. American Society of Agronomy, Madison, Wisconsin, USA. pp. 305-360).
Most silage additives are not universal but specific to the ensiling crop or problem. A silage additive is suitable for silage crops that are easy, moderately difficult or difficult to ensile, or for the inhibition of the impacts of fungi, clostridia and other microorganisms that cause spoilage of the feed.

The purpose of using silage additives is to promote the desired fermentation and inhibit the impact of undesirable microorganisms. The chemical additives restrict fermentation through acids, which increase acidity of the ensiling crop, or contain other chemicals that inhibit the actions of undesirable microorganisms. Chemical silage additives are usually used in sub-optimal ensiling conditions, while the positive impact of biological silage additives in these conditions has not been sufficiently proven. Fermentation is promoted/enhanced by biological silage additives containing sugars, enzymes or lactic acid bacteria. By adding lactic acid bacteria-based silage inoculant, this beneficial bacteria becomes dominant in the plant material. The competitiveness of lactic acid bacteria is therefore increased and the fermentation process accelerated. Lactic acid produced by lactic acid bacteria quickly results in an acidic environment in the stored silage, and successful preservation of the feed. The rapid reduction in pH inhibits the action of undesirable microorganisms (including proteolytic and pathogenic microorganisms, such as clostridia, bacilli, listeria and enteropathogens) in the silage. As a result, the damaging degradation compounds of poor fermentation (such as ammonia and butyric acid) which cause reduced feed intakes and health problems in livestock, are prevented from forming. Propionic acid, a high content of acetic acid or ethanol produced by yeasts are undesirable in silage. By preventing viablity of the microorganisms that cause spoilage of silage, nutrients that can be used for animal production, are retained in the feed. As lactic acid bacteria are part of the natural plant microflora, silage additives based on lactic acid bacteria constitute natural products that are easy to use, as unlike chemical additives they do not require special handling, and will neither corrode nor harm the environment. In the aforementioned European Commission regulation, silage additives are mentioned as technological additives.
The use of lactic acid bacteria in increasing the quality of silage fermentation has been described in several patent applications and patents.
In European patent EP0369198 (J.Setälä et al., Valio 1993), a feed additive containing *Lactobacillus plantarum DSM 4904* (AIV 755) is described.
In European patent application EP0580236 (B.B. Ten et al., Duphar International Research B.V.) the strains of microorganisms *Lactobacillus plantarum CBS 342.92* and *CBS 343.92* are described. These provide a factor reducing clostridium, namely the *proteinaceous* factor. In WO 2011/159178 a strain of Lactobacillus plantarum S deposited as KKP 2021 p and its use for ensiling roughages is described. The strain has been isolated from spontaneously fermenting maize plants and is shown to synthesize lactic acid and be active against pathogenic bacteria from Salmonella and Listeria genus, E. coli and Clostridium.

In the European patent EP 1 386 543 Lactobacillus plantarum strain MiLAB393 and its use as starter culture for ensiling straw or green fodder is described. The strain has been isolated from grass silage and is shown to inhibit the growth of several microorganisms and to lower the pH of the silage.

Saarisalo E. et al. (J Appl Microbiol. 2007 Feb;102(2):327-36) describes two Lactobacillus plantarum strains (E76 and E98) that have a broad-spectrum antimicrobial activity, e.g. against enterobacteria and clostridia, and that increase the concentration of lactic acid and decrease the pH when ensiling timothy- meadow fescue forage. In European patent EP0880323 (S.P. Mann et al., Biotal Ltd 2001) the use of *Lactobacillus buchneri* (NCIMB 40788) is described suppressing yeasts and mould and also bacteria causing porosity in feed and silage.

In UK patent GB2356125 (D. Davies et al, Genus Plc, 2003), the *Lactobacillus plantarum* NCIMB 41028 and *Lactobacillus paracasei sp.paracasei* NCIMB 41029 (used in the fermentation of material difficult to ensile) are described. These strains enable decomposing fructan (composed of residues of fructose) in ensiling material that is low in carbohydrates.

In European patent application EP2312955 (publ. WO2010017568) (E.M.Binder et al, 2010), a silage additive is described that contains at least two microorganisms of the following: *Enterococcus faecium* (DSM 3530), *Lactobacillus brevis* (DSM 19456), *Lactobacillus plantarum* (DSM 19457), *Lactobacillus kefiri* (DSM 19455), *Trichosporon spec. nov.* (DSM 14153), *Trichosporon mucoides* (DSM 14156), *Trichosporon dulcitum* (DSM 14162) and *Eubacterium* (DSM 11798) and also an anorganic substance with a large internal surface area, e.g. aluminium silicate, that decreases mycotoxins in silage and also methane emissions in animals.

In USA patent US6403084 (Chan R.K-F. et al., Pioneer Hi-Bred International, Inc., 2002) the usage of homofermentative lactic bacterium *Lactobacillus* plantarum, together with heterofermentative *Lactobacillus buchneri* or *Lactobacillus brevis* and the microorganism *Enterococcus faecium,* is described regarding the increase of the aerobic stability of silage. In international patent application WO2006007395 (Charley R. C et al., Lallemand Animal Nutrition North America, 2006), the use of the microorganism *Lactobacillus diolivorans* is described in avoiding aerobic spoilage of silage.

The problem is that most silage additives are specific to the crop that is being ensiled, or to a specific problem. This means that a silage additive is suitable for silage crops that are either easy, moderately difficult or difficult to ensile, or it inhibits the actions of yeasts, mould or other microorganisms that can cause the spoilage of feed.

The strain of the microorganism offered by this invention is suitable for the ensiling of all crops, i.e. crops that are easy, moderately difficult or difficult to ensile, inhibiting the actions of microorganisms that can cause the spoilage of feed.

### DISCLOSURE OF THE INVENTION

The invention discloses the isolated microorganism *Lactobacillus plantarum* strain TAK 59 NCIMB42150, and feed, feed additive and composition comprising the said strain. The feed may be fermented feed, e.g. silage. The feed additive may be a silage additive. Suitable excipients can be included as other ingredients in the composition.
The next object of the invention is the use of the named microorganism in: accelerating the fermentation of feed, increasing the concentration of lactic acid, reducing pH, and decreasing the loss of nutrients in feed and the concentration of ammonia nitrogen and butyric acid in feed. *Lactobacillus plantarum* TAK 59 NCIMB42150 is suitable for the ensiling of a crop that is easy, moderately difficult or difficult to ensile. *Lactobacillus plantarum* TAK 59 NCIMB42150 improves lactic acid fermentation, and as a result the lactic acid produced accelerates the decrease in the pH of silage.

The invention also discloses the use of said microorganism for inhibiting the activity of proteolytic and pathogenic microorganisms in feed. For example, proteolytic and pathogenic microorganisms may comprise enteropathogens, clostridia and yeasts. Said enteropathogens are selected from the group comprising *Listeria monocytogenes, Yersinia enterocolitica, Salmonella Enteritidis, S. entericase*rovar Typhimurium, *Shigella sonnei, Escherichia coli, Enterobacter sakazakii, Staphylococcus aureus etc.* The afeoresaid clostridia may be *Clostridium tyrobutyricum, C. butyricum, C. Sporogenes etc.*
Based on investigations into antimicrobial properties, *Lactobacillus plantarum* TAK 59 NCIMB42150 inhibits the growth and action of undesirable microorganisms.

As a result of the fast and extensive decrease of pH through targeted lactic acid fermentation with *Lactobacillus plantarum* strain TAK 59 NCIMB42150, the impact of enzymes and undesirable proteolytic pathogenic microorganisms, and the production of degradation compounds produced by them, is inhibited. When using *Lactobacillus plantarum* TAK 59 NCIMB42150, the concentrations of ammonia nitrogen and butyric acid (produced by protelytic microorganisms in silage) in feed are significantly smaller compared to the control silage or silage produced with chemical silage additives. *Lactobacillus plantarum* TAK 59 NCIMB42150 also reduces the concentrations of ethanol (produced by yeast) and the concentrations of acetic acid (produced by acetic bacteria and enterobacteria in feed).

The next object of the invention is a method of prolonging the preservation of the feed by inoculation with *Lactobacillus plantarum* TAK 59 NCIMB42150. For the livestocks, good quality feed with high nutritional value has to be ensured. As in many countries with high milk production the vegetation period does not last all year, the necessary feed has to be conserved. Such conserved feed is silage, produced when fermenting plant material with anaerobic lactic acid.
In addition to grass and legume silage crops, cereals, whole-crop cereals (incl. maize), by-products (i.e. wet distillers grain, brewers' grains, pulp) of alcohol and sugar industry are also ensiled.

By inoculating *Lactobacillus plantarum* TAK 59 NCIMB42150 into plant material to be ensiled, fermentation is accelerated, by producing more lactic acid and by reducing the pH of the feed. This, in turn, will inhibit the growth and action of undesirable microorganisms in silage, decreasing the loss of nutrients in feed. If the actions of microorganisms that cause spoilage (incl. proteolytic and pathogenic microorganisms) are inhibited in the feed, when fermented in the aforementioned way, good quality feed that is stable and can be preserved in anaerobic conditions for several months is guaranteed. This is how extending the preservation of feed is possible.

Microorganism *Lactobacillus plantarum* TAK 59 NCIMB42150 is added to feed at a rate of 1x10⁵-1x10⁶ CFU/g of the fermented feed.

### DESCRIPTION OF THE STRAIN

The object of the invention, the microorganism strain *Lactobacillus plantarum* TAK 59 NCIMB42150, was isolated in Estonia (Pärnu county) in a naturally ensiled fodder legume crop (>75 per cent) silage without using additives. To determine the quantitative content of lactobacilli in the silage sample, a suspension (with descending concentrations) was made of solutions, using the decimal dilution method in physiological solution (0.9 per cent NaCl); and seeded on Rogosa agar (OXOID, U.K.), that was incubated at 37°C in an anaerobic environment (thermostat IG 150, Jouan, France) for 48 hours. Developed colonies were described, counted and the total count of microbes was determined. To describe the morphology of microbes, preparations were made using Gram's staining method, and microscopic examinations were performed. The strain was isolated, based on colony and cell morphology specific to *Lactobacillus* spp. Provisional and detailed identification followed, this is described below.

*Lactobacillus plantarum* strain TAK 59 NCIMB42150 was deposited on May 29, 2013 according to the "Deposit of Microorganisms for the purposes of patent procedure" under the Budapest Treaty in the National Collection of Industrial, Food and Marine Bacteria (NCIMB) of the United Kingdom under the number NCIMB42150.

The morphological characteristics of the culture were determined after growth in MRS agar and broth feed (OXOID). *Lactobacillus plantarum* TAK 59 NCIMB42150 is a Gram-positive rod-shaped non-spore forming bacterium, the rods are of regular shape with medium in thickness and length occurring singly or in pairs.

### Physio-biochemical properties

MRS broth (for 24-48 hours) is suitable for cultivating the microbial strain of TAK 59 NCIMB42150, after which a homogenous turbid growth occurs. After 48 hours of cultivation in MRS agar feed at 37°C in a microaerobic environment (CO₂/O₂/N₂: 10/5/85 %) the colonies are white, convex, shiny and with a regular edging with a diameter of 1-2.5 mm.
The optimal growth temperature of the strain is 37°C; the strain also replicates at 15°C, but not at 45°C. The pH of an optimal growing environment is 6.5.

The strain of *Lactobacillus plantarum* TAK 59 NCIMB42150 is catalase- and oxidase-negative, facultatively heterofermentative, it does not hydrolyse arginine and it does not produce gas on glucose fermentation.

*Lactobacillus plantarum* TAK 59 NCIMB42150 was identified, based on biochemical activity using the test kit of API 50CHL System (bioMérieux, France) kit as *Lactobacillus plantarum* (Coincidence with the type strain: *excellent,* ID %-99.9, T index -1.0).

Identification at sequencing: *Lactobacillus plantarum* (16S rRNA similarity with strain type strain: 99 %).

The fermentation profile of *Lactobacillus plantarum* TAK 59 NCIMB42150 carbohydrates is described below, based on API CHL 50. The strain ferments: L-arabinose, ribose, D-galaktose, D-glycose, D-fructose, D-mannose, D-mannitol, D-sorbitol, methyl-αD-mannopyranosid, N-acetyl-glycosamine, amygdalin, arbutin, aeskulin, salicin, cellobiosis, maltose, D-lactose, D-melibiose, D-sucrose, D-trehalose, D-melecitosa, D-raffinose, gentibiosis, D-turanosis, K-glyconate.

### Antibiotic resistance

Method: The sensitivity of *Lactobacillus plantarum* TAK 59 NCIMB42150 to antibiotics was tested with the E-test (AB Biodisk, Solna). The minimal inhibiting concentration was determined according to the recommended epidemiological cut-off values of the European Food Safety Authority (EFSA).

**Table 1. Antibacterial sensitivity of Lactobacillus plantarum TAK 59 NCIMB42150.**

| Antibiotics | Cut-off value of *Lactobacillus plantarum* TAK 59 (mg/L) | Reference values* |
|---|---|---|
| Ampicillin | 0,047 | 2 |
| Gentamycin | 2 | 16 |
| Streptomycin | 16 | 64 |
| Erythromycin | 0,19 | 1 |
| Clindamycin | 0,5 | 1 |
| Tetracycline | 6 | 8 |
| Cloramphenicol | 3 | 4 |
| Kanamycine | 32 | 32 |

| | | |
|---|---|---|
| *Guidance on the assessment of bacterial susceptibility to antimicrobials of human and veterinary importance EFSA Journal 2012;10(6):2740 | | |

The microbial strain is considered to be sensitive when its growth at either equal or lower concentration inhibits from the cut-off value a specific antimicrobial compound (S ≤ x mg/L).

The microbial strain is considered to be resistant when its growth at higher concentration inhibits from the cut-off value a specific antimicrobial compound (R > x mg/L).

The strain of *Lactobacillus plantarum* TAK 59 NCIMB42150 showed no resistance to the antibiotics investigated (Table 1).

### FUNCTIONAL PROPERTIES OF THE STRAIN

### Profile of short-chain fatty acids

Method: The 24h old strain of *Lactobacillus plantarum* TAK59 NCIMB42150 cultivated on MRS agar was suspended in a physiological saline according to the McFarland standard at 10⁹ microbes/ml, 0.5 ml was seeded onto PYG (at 4.65 ml) and microaerobically incubated (10 % CO₂) in thermostat at 37°C for 24 h and 48 h.

The profile of short-chain fatty acids was determined by the gas chromatograph HP 6890 Series GC System, and the capillary column HP-INNOWax (15 m ×0.25 mm; 0.15 µm) was used. The programme of the column temperature was 60°C for 1 min, 20°C/min 120°C 10 min, detector (FID) 250°C (Table 2).

**Table 2. The concentration (g/l) of acetic, lactic and succinic acids in PYG of the microaerobic cultivation of Lactobacillus plantarum TAK 59 NCIMB42150 for 24 h and 48 h**

| | Short-chain fatty acids (g/l) | | | | | |
|---|---|---|---|---|---|---|
| | Acetic acid | | Lactic acid | | Succinic acid | |
| | 24 h | 48 h | 24h | 48h | 24h | 48h |
| *L.plantarum* TAK 59 | 0.081 | 0.131 | 1.244 | 1.352 | 0.061 | 0.069 |

Antimicrobial activity towards lactobacillus and pathogens of plant origin

To evaluate the antimicrobial properties towards pathogens, the pour plate technique was used (Hutt P, Shchepetova J, Loivukene K, Kullisaar T, Mikelsaar M. Antagonistic activity of probiotic lactobacilli and bifidobacteria against entero- and uropathogens. J Appl Microbiol. 2006; 100(6):1324-32).

The target microbe growth inhibition zone in millimetres was measured in streak-line procedure. Similarly, the arithmetical mean and standard error (Table 3) were calculated based on the results of the sample used according to Hütt et al (2006); thus the antagonistic activity of strains was evaluated as follows:
Weak <20.9; average 21.0-23.9; strong >24; inhibition zone in anaerobic environment (mm-s): weak <20.9; average 21.0-22.9; strong >23.

**Table 3. The antimicrobial activity of Lactobacillus plantarum TAK59 NCIMB42150 against lactobacilli of plant origin and pathogens, using the streak-line method on modified MRS agar medium (target microbe growth inhibition zone in mm) in microaerobic (10% CO₂) and anaerobic (CO₂/N₂/H₂: 5/90/5 %) environments**

| Pathogen | Growth reduction zone (mm) | |
|---|---|---|
| | Microaerobic | Anaerobic |
| *Lactobacillus* spp | 9.9 ± 2.3 | 16.1± 1.3 |
| *Listeria monocytogenes* | 22.9± 1.83 | 21.17±1.33 |
| *Yersinia enterocolitica* | 27.2± 2.46 | 24.25±2.06 |
| *Salmonella enteritidis* | 23.25± 1.06 | 22.7±0.51 |
| *S. enterica* serovar Typhimurium | 21.87± 1.45 | 21.5±0.55 |
| *Shigella sonnei* | 16.93± 2.46 | 23.0±1.41 |
| *Escherichia coli* | 23.12± 1.41 | 21.8±0.75 |
| *Enterobacter sakazakii* | 23.3± 0.95 | 22.5±1.22 |
| *Staphylococcus aureus* | 22.93±2.01 | 22.67±1.5 |
| *Enterococcus faecalis* | 21.81±1.87 | 21.16±0.98 |

Inhibition zone in microaerobic environment (mm-s): weak <20.9; average 21.0-23.9; strong >24.

Inhibition zone in anaerobic environment (mm-s): weak <20.9; average 21.0-22.9; strong >23.

The antimicrobial activity towards clostridia was determined as follows.

Supernatant was isolated from the 24h old *Lactobacillus plantarum* TAK59 NCIMB42150 culture in the Brain Heart Infusion (BHI) broth. The suspension of clostridia was added into the sterilised (by filtration) supernatant or into the BHI broth (positive check). The results were evaluated after 48h at OD₆₂₀ₙₘ. The growth inhibition of clostridia CD (%) was calculated as follows = 100 - (ODₜ X 100 / ODₜ), of which
ODₜ - supernatant added
OD_{c} - no supernatant added

The antimicrobial compounds produced by *Lactobacillus plantarum* TAK59 NCIMB42150 inhibited the decrease of plant-origin clostridia by 19.81 per cent.

### DESCRIPTION OF THE DRAWINGS

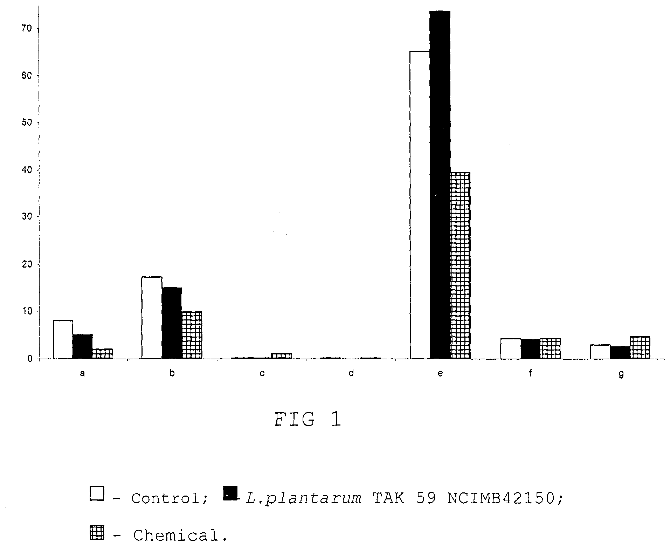

Fig. 1 - Improving the fermentation quality of easy to ensile forage with *L.plantarum* strain TAK 59 NCIMB42150, where a - ethanol, g/kg dry matter; b - acetic acid, g/kg dry matter; c - propionic acid, g/kg dry matter; d - butyric acid, g/kg dry matter; e - lactic acid, g/kg dry matter; f - pH; g - ammonia nitrogen in total nitrogen, per cent;

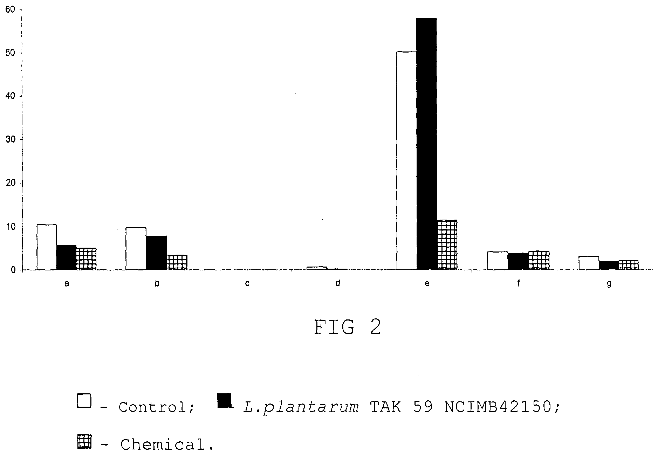

Fig. 2 - Improving the fermentation quality of moderately difficult to ensile forage with *L.plantarum* strain TAK 59 NCIMB42150, where a - ethanol, g/kg dry matter; b - acetic acid, g/kg dry matter; c - propionic acid, g/kg dry matter; d - butyric acid, g/kg dry matter; e - lactic acid, g/kg dry matter; f - pH; g - ammonia nitrogen in total nitrogen, per cent;

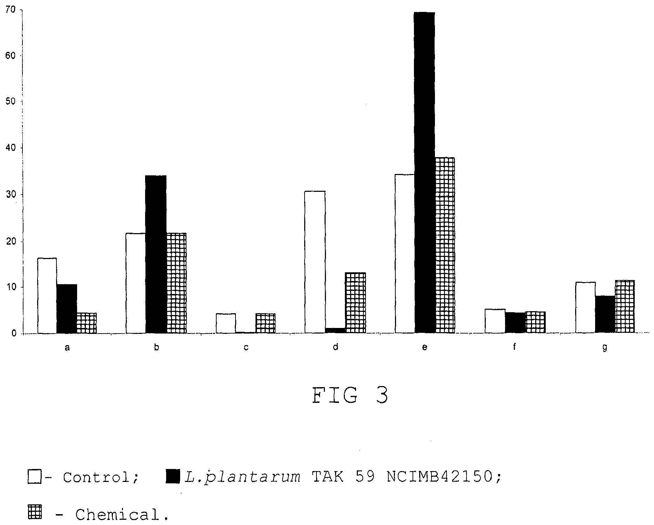

Fig. 3 - Improving the fermentation quality of difficult to ensile forage with *L.plantarum* strain TAK 59 NCIMB42150, where a - ethanol, g/kg dry matter; b - acetic acid, g/kg dry matter; c - propionic acid, g/kg dry matter; d - butyric acid, g/kg dry matter; e - lactic acid, g/kg dry matter; f - pH; g - ammonia nitrogen in total nitrogen, per cent.

### DESCRIPTION OF THE EMBODIMENTS

Example 1. Improving the fermentation quality of plant material easy to ensile forage with *Lactobacillus plantarum* TAK 59 NCIMB42150.

The test was made with a mixture of red clover (*Trifolium pratense* L.) and timothy (*Phleum pratense* L.) forage, at a ratio of 25:75. The content of water soluble carbohydrates in the ensiling material was 3.01 per cent. Fresh forage was cut and wilted for 48h. The wilted forage was harvested, chopped and the control silage was prepared. The control silage was made without silage additives.

*Lactobacillus plantarum* strain TAK 59 NCIMB42150 was inoculated into the second silage, and the third silage was prepared with a chemical silage additive with the following content: formic acid 42.5 per cent, ammonium formate 30.3 per cent, propionic acid 10.0 per cent, benzoic acid 1.2 per cent, ethyl benzoate 1.0 per cent and water 15.0 per cent. The *Lactobacillus plantarum* strain TAK 59 NCIMB42150 was inoculated into the ensiled forage at a concentration of 1.2x10⁵ CFU/g of the ensiled fresh plant material (feed). The test silages were opened after 100 days of ensiling.

The samples were analysed according to generally accepted methods (AOAC, 2005. Official methods of analysis of AOAC International, 18th ed. Association of Official Analytical Chemists International, Gaithersburg, MD, USA).

For determination of the dry matter content, the silage sample was dried in a thermostat at 130 °C to constant weight. For determination of the crude ash content , the silage sample was ashed in a muffle furnace for 6h at 550 °C. Protein content was determined with a Kjeltec™ 2300 analyser, using the Kjeldhal method (Nx6.25). For the determination of neutral detergent fibre (NDF) and acid detergent fibre (ADF), the method described by Van Soest et al was used (Van Soest, P. J., Robertson, J. B., Lewis, B. A. 1991. Methods for dietary fiber, neutral detergent fiber and nonstarch polysaccharides in relation to animal nutrition - J. Dairy Sci. 74: 3583-3597) and fibre analyser ANKOM²²⁰. Gas chromatograph Agilent 7890A was used for determination of the acid and ethanol contents of the silage. The ammonia nitrogen content in total nitrogen was determined with a Kjeltec™ 2300 analyser. The pH of the silage was determined with a pH-metre, Hanna Instruments pH 210.

Compared to the control silage, the microorganism *Lactobacillus plantarum* TAK 59 NCIMB42150 improved the fermentation of the silage (see Table 4 and Fig. 1). Inoculation with *Lactobacillus plantarum* strain TAK 59 NCIMB42150 increased the lactic acid content, and the pH decreased to 4.1. Hence the growth and action of undesirable micro-organisms in the silage was inhibited, which was expressed through a smaller content of ethanol (produced by yeast), and of acetic acid (produced by acetic bacteria and enteropathogens). Silage made using

*Lactobacillus plantarum* TAK 59 NCIMB42150 also had lower ammonia nitrogen and butyric acid contents (produced by proteolytic microorganisms).

Despite the easy ensiling conditions of the aforementioned example, the fermentation quality parameters of silage (by way of lactic bacteria natural population) were poorer than those of the silage with the additive *Lactobacillus plantarum* TAK 59.

Hence, *Lactobacillus plantarum* TAK 59 NCIMB42150 improved the fermentation of crop silage that was easy to ensile, and therefore improved the quality of the feed. The more extensive decrease in pH inhibited the growth and action of undesirable proteolytic microorganisms and enteropathogens that cause spoilage, and therefore reduced the loss of nutrients in feed. In feed fermented in this way, the actions of microorganisms that cause spoilage are inhibited, the pH remains low and the feed remains in an anaerobic condition for several months.

Example 2. Improving the fermentation quality of moderately difficult to ensile forage with *Lactobacillus plantarum* strain TAK 59 NCIMB42150.

The test was made with a mixture of meadow fescue (*Festuca pratensis*) and timothy (*Phleum pratense* L.) forage of, at a ratio of 50:50. Fresh forage was cut and wilted for 48h. The wilted forage was harvested, chopped and a test silage was prepared. The of water soluble carbohydrates content in the ensiling material was 2.15 per cent. The control silage was made without silage additives. *Lactobacillus plantarum* strain TAK 59 NCIMB42150 was inoculated into the second silage, and the third silage was prepared with a chemical silage additive with the following content: formic acid 76.0 per cent, ammonium formate 5.5 per cent, water 18.5 per cent. *Lactobacillus plantarum* strain TAK 59 NCIMB42150 was inoculated into the ensiled forageat a concentration of 2.4x10⁵ CFU/g of the fresh plant material. The test silages were opened after 90 days of ensiling.

Silage samples were analysed according to the methods described in the first example above.

*Lactobacillus plantarum* TAK 59 NCIMB42150 improved the quality of silage from a fermenting crop that is moderately difficult to ensile (Table 5 and Fig. 2). With dry matter content of ca 36 per cent, with *Lactobacillus plantarum* TAK 59 NCIMB42150 the pH level was 3.9; the corresponding parameter for silage without silage additive was pH 4.2. The pH of silage is directly connected to the content of lactic acid, hence the silage prepared with *Lactobacillus plantarum* strain TAK 59 NCIMB42150 had a higher production of lactic acid and also a lower pH level. This means that inoculation with *Lactobacillus plantarum* strain TAK 59 NCIMB42150 accelerated lactic acid fermentation in feed, and the production of fermentation products that might appear in association with the undesirable fermentation of silage was prevented. During faster fermentation, the loss of nutrients was smaller. Compared to the control silage, inoculation with *Lactobacillus plantarum* TAK 59 NCIMB42150 decreased the ethanol, acetic acid, butyric acid and ammonia-nitrogen contents in silage. The butyric acid content decreased by 5.2 times and ammonia and ethanol by 1.5 and 1.8 respectively.

Therefore, *Lactobacillus plantarum* TAK 59 NCIMB42150 improved the fermentation of crop silage that was moderately difficult to ensile, hence improving the quality of feed. The more extensive reduction in pH inhibited the growth and action of undesirable proteolytic microorganisms and enteropathogens that cause spoilage, therefore decreasing the loss of nutrients in feed. In feed fermented in this way, the actions of microorganisms that cause spoilage are inhibited, pH remains low and stable and the quality of the feed remains in an anaerobic condition for several months.

Example 3. Improving the fermentation quality of difficult to ensile forage with *Lactobacillus plantarum* TAK 59 NCIMB42150.

The silage additive *Lactobacillus plantarum* TAK 59 NCIMB42150 also works in difficult ensiling conditions, and a test using a mixture of a red clover (*Trifolium pratense* L.) and timothy (*Phleum pratense* L.) forage was made to demonstrate this. Their corresponding ratio was 25:75. Fresh forage was harvested, chopped and test silages were prepared. The water soluble carbohydrates content in the harvested material was 0.91 per cent. The control silage was made without silage additives, the *Lactobacillus plantarum* strain TAK 59 NCIMB42150 was inoculated into the second silage, and the third silage was prepared with a chemical silage additive with the following content: formic acid 42.5 per cent, ammoniumformate 30.3 per cent, propionic acid 10.0 per cent, benzoic acid 1.2 per cent, ethyl benzoate 1.0 per cent, water 15.0 per cent. *Lactobacillus plantarum* TAK 59 strain NCIMB42150 was inoculated into the ensiled crop at a concentration of 1.2x10⁵ CFU/g of fresh material (feed). The test silages were opened after 100 days of ensiling.

Silage samples were analysed according to the methods described in the first example above.

Forage difficult to ensile is characterised by low dry matter and sugar content, and also by the fact that silage prepared without silage additive shows very poor fermentation parameters (Table 6 Fig. 3). The control silage contained a lot of butyric acid and ammonia nitrogen, which indicates extensive proteolysis during fermentation. In the control silage, the butyric and lactic acid contents were approximately the same, of which the former was considered to be too high and the latter too low; such silage cannot be fed to animals. On the other hand, the silage prepared with *Lactobacillus plantarum* TAK 59 NCIMB42150 comprised twice as much lactic acid and the butyric acid content (produced by clostridia) was insignificant. When ensiling forage that is difficult to ensile, *Lactobacillus plantarum* TAK 59 NCIMB42150 reduced the pH of the silage to 4.4, whereas the pH of the control silage was 5.1. Similarly, when compared to the control silage, the silage prepared with *Lactobacillus plantarum* TAK 59 NCIMB42150 had better parameters for ammonia nitrogen, propionic acid and ethanol. Silages inoculated with lactic acid bacteria TAK 59 NCIMB42150 were uniform in terms of quality, whereas the corresponding parameters of the control silage greatly varied. Unlike the control silage, the pH of the silage prepared with *Lactobacillus plantarum* TAK 59 NCIMB42150 decreased faster during fermentation, and to a greater extent, and the growth and action of undesirable microorganisms was inhibited, and the loss of nutrients was diminished in the silage.

Therefore, *Lactobacillus plantarum* TAK 59 NCIMB42150 improved the fermentation of forage silage that is difficult to ensile, and therefore improved the quality of the feed. The more extensive reduction in pH inhibited the growth and action of the undesirable proteolytic microorganisms and enteropathogens which cause spoilage, and thus reduced the loss of nutrients from feed. In feed fermented in this way, the action of the microorganisms that cause spoilage are inhibited, pH remains low and stable and the quality of the feed remains in an anaerobic condition for several months.

In all three examples, the pH and parameters of lactic acid and ammonia nitrogen in silages prepared with *Lactobacillus plantarum* TAK 59 NCIMB42150 were also better than those prepared with the chemical silage additives. Once more, this indicates the effectiveness of *Lactobacillus plantarum* TAK 59 NCIMB42150 when ensiling silage crops at different levels of ensiling difficulty.

**Table 4. Improving the fermentation quality of easy to ensile forage with Lactobacillus plantarum TAK 59 NCIMB42150.**

| Parameter | Control | | TAK 59 | | Chemical | |
|---|---|---|---|---|---|---|
| | Mean | SD | Mean | SD | Mean | SD |
| Dry matter, g/kg | 381.9 | 0.8 | 387.8 | 6.4 | 378.2 | 4.1 |
| In dry matter, g/kg | | | | | | |
| Crude protein | 157.9 | 1.8 | 159.3 | 1.1 | 168.0 | 1.8 |
| Crude ash | 96.6 | 0.3 | 93.3 | 1.4 | 94.1 | 0.3 |
| NDF | 490.3 | 5.7 | 488.3 | 4.3 | 472.7 | 6.5 |
| ADF | 356.6 | 4.3 | 358.3 | 13.6 | 343.1 | 2.3 |
| Ethanol | 8.27 | 0.25 | 5.17 | 0.40 | 2.13 | 0.06 |
| Acetic acid | 17.30 | 0.36 | 15.07 | 0.32 | 9.87 | 0.67 |
| Propionic acid | 0.10 | 0.00 | 0.10 | 0.00 | 1.07 | 0.12 |
| Butyric acid | 0.10 | 0.00 | 0.03 | 0.06 | 0.10 | 0.00 |
| Lactic acid | 65.03 | 2.56 | 73.70 | 1.30 | 39.33 | 0.93 |
| pH | 4.3 | 0.02 | 4.1 | 0.01 | 4.4 | 0.02 |
| NH₃-N/total N, % | 3.03 | 0.06 | 2.70 | 0.00 | 4.83 | 0.15 |

**Table 5. Improving the fermentation quality of moderately difficult to ensile forage with Lactobacillus plantarum TAK 59 NCIMB42150.**

| Parameter | Control | | TAK 59 | | Chemical | |
|---|---|---|---|---|---|---|
| | Mean | SD | Mean | SD | Mean | SD |
| Dry matter, g/kg | 367.3 | 3.7 | 363.9 | 1.0 | 376.5 | 0.8 |
| In dry matter, g/kg | | | | | | |
| Crude protein | 98.6 | 0.5 | 97.9 | 2.9 | 95.0 | 2.3 |
| Crude ash | 72.6 | 0.9 | 72.5 | 0.4 | 69.8 | 1.2 |
| NDF | 639.0 | 4.6 | 633.6 | 14.5 | 620.3 | 2.0 |
| ADF | 405.4 | 5.0 | 398.1 | 10.0 | 401.1 | 10.3 |
| Ethanol | 10.47 | 1.04 | 5.67 | 0.40 | 5.10 | 0.44 |
| Acetic acid | 9.90 | 0.44 | 7.87 | 0.47 | 3.47 | 0.06 |
| Propionic acid | 0.00 | 0.00 | 0.03 | 0.06 | 0.00 | 0.00 |
| Butyric acid | 0.67 | 0.29 | 0.13 | 0.06 | 0.07 | 0.06 |
| Lactic acid | 50.13 | 1.06 | 57.90 | 5.43 | 11.43 | 1.62 |
| pH | 4.2 | 0.00 | 3.9 | 0.00 | 4.4 | 0.15 |
| NH₃-N/total N, % | 3.03 | 0.15 | 1.97 | 0.12 | 2.10 | 0.10 |

**Table 6. Improving the fermentation quality of difficult to ensile forage with Lactobacillus plantarum TAK 59 NCIMB42150.**

| Parameter | Control | | TAK 59 | | Chemical | |
|---|---|---|---|---|---|---|
| | Mean | SD | Mean | SD | Mean | SD |
| Dry matter, g/kg | 132.7 | 5.0 | 136.4 | 1.2 | 138.5 | 5.9 |
| In dry matter, g/kg | | | | | | |
| Crude protein | 153.1 | 8.3 | 171.0 | 2.1 | 177.2 | 3.3 |
| Crude ash | 97.4 | 3.0 | 93.2 | 1.1 | 89.2 | 3.5 |
| NDF | 544.4 | 23.5 | 499.7 | 4.1 | 517.1 | 23.2 |
| ADF | 383.3 | 18.1 | 362.2 | 8.3 | 362.3 | 10.7 |
| Ethanol | 16.23 | 1.81 | 10.53 | 0.46 | 4.43 | 1.00 |
| Acetic acid | 21.57 | 6.79 | 34.03 | 0.68 | 21.67 | 5.62 |
| Propionic acid | 4.23 | 3.48 | 0.13 | 0.06 | 4.23 | 3.00 |
| Butyric acid | 30.53 | 26.33 | 0.93 | 0.12 | 13.03 | 12.21 |
| Lactic acid | 34.17 | 31.87 | 69.27 | 6.75 | 37.73 | 9.89 |
| pH | 5.1 | 0.4 | 4.4 | 0.1 | 4.6 | 0.4 |
| NH₃-N/total N, % | 10.97 | 1.93 | 7.97 | 0.25 | 11.40 | 4.00 |

## Claims

1. An isolated microorganism strain *Lactobacillus* plantarum TAK 59 NCIMB42150.

2. The microorganism strain of claim 1 in lyophilised form.

3. The feed comprising the microorganism strain of any of claims 1 and 2.

4. The feed of claim 3, in the form of fermented feed, e.g. silage.

5. A composition comprising the microorganism strain of claim 1.

6. Use of microorganism strain of claim 1 as a feed additive.

7. Use of microorganism strain of claim 1 in the fermentation of feed.

8. Use of microorganism strain of claim 1 to accelerate the fermentation of feed, to increase the concentration of lactic acid in feed, to reduce pH and hence decrease the loss of nutrients in feed and decrease ammonia nitrogen and butyric acid concentrations in feed.

9. Use of microorganism strain of claim 1 in inhibiting the activity of proteolytic and pathogenic microbes, by adding the microorganism strain of claim 1 to the feed to be fermented.

10. Use according to claim 9, where proteolytic and pathogenic microbes are clostridia and enteropathogens.

11. Method for prolonging the preservation of feed, where the microorganism of claim 1 is inoculated/added to the feed to be fermented.

## Patentansprüche

1. Isolierter Mikroorganismenstamm *Lactobacillus* plantarum TAK 59 NCIMB42150.

2. Mikroorganismenstamm nach Anspruch 1 in lyophilisierter Form.

3. Futter welche Mikroorganismenstamm nach einem der vorangehenden Ansprüche 1 und 2 enthält.

4. Futter nach Anspruch 3, in Form von fermentiertem Futter, z.B. Silage

5. Zusammensetzung, welche Mikroorganismusstamm nach Anspruch 1 enthält.

6. Verwendung des Mikroorganismenstamms nach Anspruch 1 als Futterzusatzstoff.

7. Verwendung des Mikroorganismenstamms nach Anspruch 1 für Fermentation von Futter.

8. Verwendung des Mikroorganismenstamms nach Anspruch 1 zur Beschleunigung der Fermentation von Futter, zur Erhöhung der Konzentration von Milchsäure im Futter, zur Reduzierung des pH-Werts und damit zur Verringerung des Nährstoffverlusts im Futter und zur Verringerung der Ammoniakstickstoff- und Buttersäurenkonzentration im Futter.

9. Verwendung eines Mikroorganismenstamms nach Anspruch 1 zur Hemmung der Aktivität von proteolytischen und pathogenen Mikroben durch Zugabe des Mikroorganismenstamms nach Anspruch 1 zu dem zu fermentierenden Futter.

10. Verwendung nach Anspruch 9, wobei proteolytische und pathogene Mikroben Clostridien und Enteropathogen sind.

11. Verfahren zur Verlängerung der Haltbarkeit von Futter, wobei der Mikroorganismus nach Anspruch 1 in den zu fermentierenden Futter eingeimpft / hinzugefügt wird.

## Revendications

1. La souche isolée de microorganisme *Lactobacillus plantarum* TAK 59 NCIMB42150.

2. La souche de microorganisme de la revendication 1 dans forme lyophilisée.

3. Les aliments pour animaux comprenant la souche de microorganisme selon l'une quelconque des revendications 1 et 2.

4. Les aliments pour animaux selon la revendication 3, sous forme d'alimentation fermentée, par exemple sous formed'un ensilage.

5. La composition comprenant la souche de microorganisme selon la revendication 1.

6. L'utilisation de la souche de microorganisme selon la revendication 1 en tant qu'additif alimentaire.

7. L'utilisation de la souche de microorganisme selon la revendication 1 dans la fermentation des aliments pour animaux.

8. L'utilisation de la souche de microorganisme selon la revendication 1 pour accélérer la fermentation des aliments pour animaux, pour augmenter la concentration d'acide lactique dans les aliments pour animaux, pour réduire le pH et diminuer ainsi la perte de nutriments dans les aliments pour animaux, ainsi que pour diminuer les concentrations d'azote de l'ammoniac et d'acide butyrique dans les aliments pour animaux.

9. L'utilisation d'une souche de microorganisme selon la revendication 1 pour inhiber l'activité des microbes protéolytiques et pathogènes, en ajoutant aux aliments à fermenter la souche de microorganisme selon la revendication 1.

10. L'utilisation selon la revendication 9, dans laquelle les microbes protéolytiques et pathogènes sont des clostridies et des enteropathogènes.

11. Le procédé pour prolonger la conservation des aliments pour animaux, dans lequel le microorganisme de la revendication 1 est inoculé / ajouté aux aliments à fermenter.
